# EUROPÄISCHE PATENTSCHRIFT

(11) **EP 1 679 044 B1**
(45) Veröffentlichungstag und Bekanntmachung des Hinweises auf die Patenterteilung: **08.04.2009**
(21) Anmeldenummer: 05023224.8
(22) Anmeldetag: 25.10.2005
(51) Int. Cl.: A61B 17/80, A61B 17/86, A61B 17/68

(54) **Set zur Erstellung eines Osteosyntheseimplantates**
Set for creation of an implant for osteosynthesis
Set pour la création d'un implant d'ostéosynthèse

(30) Priorität: 07.01.2005 DE 102005001438
(43) Veröffentlichungstag der Anmeldung: 12.07.2006
(73) Patentinhaber: Olms, Kai-Hinrich, Dr., 23611 Bad Schwartau (DE)
(72) Erfinder: Nassut, Roman, Dr., 23879 Mölln (DE); Olms, Kai-Hinrich, Dr., 23611 Bad Schwartau (DE)
(74) Vertreter: Vollmann, Heiko

(56) Entgegenhaltungen:
- EP-A- 0 355 035
- WO-A-99/09903
- DE-U1- 9 308 944
- US-A1- 2002 143 338
- US-A1- 2003 125 743
- PATENT ABSTRACTS OF JAPAN Bd. 2002, Nr. 07, 3. Juli 2002 (2002-07-03) -& JP 2002 065682 A (DEPUY JAPAN INC), 5. März 2002 (2002-03-05)

## Beschreibung

Die vorliegende Erfindung betrifft ein Set zur Erstellung eines Osteosyntheseimplantates.

Üblich ist der Einsatz von sogenannten Osteosyntheseplatten, meist mit der Möglichkeit zur Längeneinstellung einer Osteotomie, wie sie beispielsweise bekannt ist aus der DE-C-40 07 306. Grundsätzlich stellt sich bei Knochenfrakturen das Problem ein, die Knochenteile unter exakter Ausrichtung zueinander zu fixieren. Bei anderen Indikationen wird zunächst gezielt ein Knochen frakturiert, um hernach die beiden Knochenteile an ihrer Trennstelle (Osteotomie) in einem festen oder aber variabel gehaltenen Abstand zueinander zu fixieren, so dass die Knochenteile Gelegenheit erhalten, wieder zusammen zu wachsen.

Die bekannte Osteosyntheseplatte besteht aus zwei gegeneinander längs verschieblichen Plattenteilen, die mit den Knochenteilen fixierbar sind, beispielsweise durch Knochenschrauben. Die mechanische Führung bei der bekannten Osteosyntheseplatte ist gebildet durch eine Schwalbenschwanzführung. In deren Bereich ist ein extrakorporal betätigbarer Getriebemechanismus vorgesehen, mit dem die Plattenteile zueinander längs verschoben werden können. Hierzu ist der Getriebemechanismus so ausgebildet, dass eine Welle beispielsweise eine solche Länge und Orientierung aufweist, dass sie nach der Implantation in den Patientenkörper an nur einer einzigen Stelle die Haut des Patienten durchsticht. Von außen her lässt sich dann die Welle mittels eines geeigneten Werkzeuges, beispielsweise eines Schraubenschlüssels, so bedienen, dass die Osteotomie verlängert oder verkürzt werden kann.

Wenn die bekannte Osteosyntheseplatte in der Praxis auch gute Ergebnisse liefert, so birgt ihr dennoch ein gravierender Nachteil inne, wenn sie nämlich vor allem im Kiefer- und Schädelbereich zum Einsatz kommen soll. Aufbaubedingt nämlich trägt die bekannte Osteosyntheseplatte relativ weit auf, d. h. ihre räumliche Tiefe ist relativ groß. Dies ist zurückzuführen auf die Ausbildung der mechanischen Führung als Schwalbenschwanzführung. Bei einer naturgemäß vorgegebenen räumlichen Enge am Implantationsort ist dies äußerst unzweckmäßig.

Mit der Osteosyntheseplatte gemäß der DE-C-195 42 064 ist versucht worden, dass diese aufgrund ihres konstruktiven Aufbaus wesentlich geringere Aufbaumaße aufweist, um so an Implantationsorten mit einer räumlichen Enge zum Einsatz kommen zu können, also beispielsweise im Kiefer- oder Schädelbereich.

Es ist daher vorgeschlagen worden, dass die mechanische Führung aus wenigstens zwei an dem ersten Plattenteil angeformten und in Längsverschieberichtung orientierten zylindrischen Zapfen und wenigstens zwei in dem zweiten Plattenteil vorgesehenen, in Längsverschieberichtung orientierten zylindrischen Bohrungen besteht, wobei die Zapfen in den Durchbohrungen gelagert sind.

Das Dokument JP 2002 065682 A offenbart ein Set aufweisend eine Platte und zwei mit zwei Zugankern verschraubbare Gewindeelemente.

Die bekannten Osteosynthesplatten beschäftigen sich also in erster Linie mit der Längsverschieblichkeit einer Osteotomie. Sie können Anwendung finden bei Osteotomien oder Frakturen von relativ großen Röhrenknochen. Ein Einsatz bei kleineren Knochen, beispielsweise im Fußbereich, ist praktisch nicht möglich aufgrund der immer noch erheblichen Baugröße. Darüber hinaus stellen sie mechanisch gesehen oft ein instabiles System dar mit unvorhersehbaren Biegemomenten, die in ermündender Weise auf das Material der Osteosyntheseplatten einwirken, sowie mit unvorhersehbaren Kräften, die auf die der Fixation der Platten dienenden Knochenschrauben wirken. All dies führt oft zu Brüchen der bekannten Osteosynthesplatten.

Vor dem aufgezeigten Hintergrund ist es nun die Aufgabe der vorliegenden Erfindung, ein Set zur Erstellung eines Osteosyntheseimplantates vorzuschlagen, welches im Bereich relativ kleiner Knochen, beispielsweise im Fuß- oder auch Handbereich, Anwendung finden kann und zum anderen ein mechanisch vorhersehbar belastbares System darstellt, so dass Ermüdungsbrüche etc. nicht zu erwarten sind.

Gelöst wird diese Aufgabe durch ein Set mit den Merkmalen des Anspruchs 1, welches weitergebildet wird mit den Merkmalen der Unteransprüche.

Demgemäß wird ein Set zur Erstellung eines Osteosyntheseimplantates vorgeschlagen, welches aufweist:
- ein plattenförmiges Grundelement mit wenigstens zwei Durchbohrungen,
- wenigstens zwei stabförmige Zuganker, die mit ihrem einen ersten Ende in die Durchbohrungen setzbar sind, wobei die Durchbohrungen und die ersten Enden der Zuganker derart ausgebildet sind, dass die Enden in den Durchbohrungen verriegelbar sind, wobei die Zuganker im verriegeltem Zustand eine Schwenkbewegung ausführen können, sowie
- wenigstens zwei Gewindeelemente, die mit den mit jeweils einem Gewinde versehenen zweiten Enden der wenigstens zwei Zuganker verschraubbar sind.

Die Verschwenkbarkeit der Zuganker spielt, wenn diese in den Durchbohrungen in der Platte verriegelt sind, im implantierten Zustand keine Rolle mehr. Die Verschwenkbarkeit ist aber Voraussetzung für eine spannungsfreie Implantation, da sie es nämlich gestattet, Durchbohrungen im Knochen in unterschiedlichen Richtungen einzubringen, je nach patientenindividuellen Gegebenheiten, die in situ vom Operateur berücksichtigt werden können. Operativ geht man nun so vor, dass das plattenförmige Grundelement zunächst unter die Osteotomie bzw. Fraktur mittels einer Spannzange unterklemmt wird. Sodann bringt der Operateur die Bohrungen im Knochen an, und zwar - wie ausgeführt - je nach geeigneten Gegebenheiten. Sodann werden die wenigstens zwei Zuganker, die mit ihren zweiten Enden mit den wenigstens zwei Gewindeelementen verschraubt sind, von der anderen Seite des Knochens in die Bohrungen im Knochen gesetzt. Die Längenverhältnisse sind dabei so gewählt, dass die ersten Enden der Zuganker auf jeden Fall bis zu den Durchbohrungen reichen und dort eingesetzt werden können. Eine weitere Betätigung der Gewindeelemente führt zu der besagten Verriegelung. In situ ist also die Verschwenkbarkeit der Zuganker in den Durchbohrungen kein Erfordernis mehr. Sie ist vielmehr ein Merkmal des Implantates, das aus dem Set erstellt werden kann, vor der Implantation.

Der Vorteil des erfindungsgemäßen Sets ist darin zu sehen, dass die Neigungen der Knochenbohrungen patientenindividuell eingestellt werden können, und zwar so, dass das plattenförmige Grundelement und die Zuganker einschließlich der Gewindeelemente ohne unerwünschte zusätzliche Zugkräfte, Biegemomente etc. implantiert werden können. Gewissermaßen der Preis hierfür ist, dass das plattenförmige Grundelement Biegemomente in nur einer Richtung aufnehmen kann, dies aber definiert und mechanisch vorhersehbar. Die entlang der Zuganker erzeugten Zugkräfte sind erwünscht, da diese für einen sicheren Halt des Implantates am Knochen sorgen.

Das Osteosyntheseimplantat, welches aus dem erfindungsgemäßen Set erstellt werden kann, eignet sich hervorragend für den Einsatz in der Fuß- aber auch in der Handchirurgie bei beengten Raumverhältnissen und kleinen Knochen. Das erfindungsgemäße Implantat ist nicht konzipiert für den Einsatz an großen Röhrenknochen, wie beispielsweise dem Femur oder der Tibea, wo sehr viel größere Belastungen auftreten und auch mit Biegemomenten in mehr als nur einer Richtung zu rechnen ist.

Gemäß einer vorteilhaften Weiterbildung ist vorgesehen, dass die Zuganker im in den Durchbohrungen verriegelten Zustand eine Schwenkbewegung um die Nulllage, in der die Zuganker senkrecht auf dem plattenförmigen Grundelement stehen, um ± 20° ausführen können. Dies wohlgemerkt nur im Auslieferungszustand und im zusammengesetzten Zustand. Wie ausgeführt, spielt diese Verschwenkbarkeit nach der Implantation keine Rolle mehr. Sie ist vielmehr Voraussetzung für die spannungsfreie Implantation am Knochen.

Gemäß einer Ausführungsform ist vorgesehen, dass die Zuganker als Gewindehülsen und die Gewindeelemente als Schrauben ausgebildet sind.

In kinematischer Umkehr kann vorgesehen sein, dass die Zuganker an ihren zweiten Enden als Gewindespindeln und die Gewindeelemente als Gewindehülsen ausgebildet sind.

Gemäß einer vorteilhaften Weiterbildung ist vorgesehen, dass die ersten Enden der Zuganker mit den Durchbohrungen im plattenförmigen Grundelement eine Art Bajonettverschluss ausbilden. Nach einer Drehung der auf den zweiten Enden der Zuganker geschraubten Gewindeelemente werden die ersten Enden der Zuganker in den Durchbohrungen verriegelt. Ein weiteres Anziehen der Gewindeelemente erhöht die Zugkräfte in den Zugankern, was bis zu einem gewissen Grade erwünscht ist, um die Osteotomie bzw. Fraktur zu stabilisieren.

Gemäß einer noch weiteren vorteilhaften Ausbildung ist vorgesehen, dass die dem Knochen zuzuwendende Fläche des plattenförmigen Grundelements zumindest abschnittsweise mit einer offenmaschigen, dreidimensionalen Raumnetzstruktur versehen ist. Diese Raumnetzstruktur ermöglicht das Ein- und Hindurchwachsen von Knochenmaterial zur Langzeitfixation.

In ähnlicher Weise kann gemäß einer vorteilhaften Weiterbildung vorgesehen sein, dass die dem Knochen zuzuwendenden Flächen der Gewindeelemente zumindest teilweise mit einer offenmaschigen, dreidimensionalen Raumnetzstruktur versehen sind, und zwar ebenfalls zur Herstellung einer stabilen Sekundärfixation. In diesem Falle ist eine zweite Operation zur Entfernung des Implantates nach Verheilung der Fraktur nicht vorgesehen.

Aus Stabilitätsgründen bestehen das Grundelement, die Zuganker und die Gewindeelemente bevorzugt aus körperverträglichem Metall.

Die Erfindung wird anhand eines Ausführungsbeispiels gemäß den Zeichnungsfiguren näher erläutert. Hierbei zeigt:
- Figur 1: eine Schnittansicht durch die drei Komponenten des Sets,
- Figur 2: eine schematische Ansicht des Grundelements mit einem Zuganker,
- Figur 3: eine Aufsicht auf das plattenförmige Grundelement sowie die Vergrößerung der Einzelheit Z,
- Figur 4: eine vergrößerte Schnittansicht durch das Grundelement im Bereich einer der Durchbohrungen mit eingesetztem und verriegeltem Zugankerund
- Figur 5: schematisch das aus dem Set erstellte Osteosyntheseimplantat nach der Implantation.

Nachfolgend sind gleiche Teile mit denselben Bezugszeichen versehen.

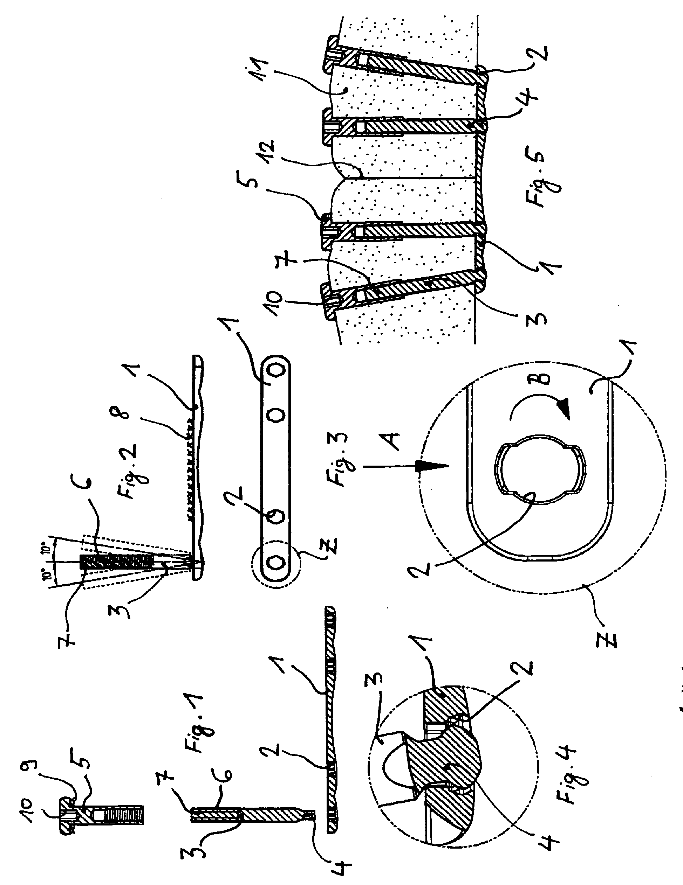

Einen ersten Überblick verschafft Figur 1. Diese zeigt einen schematischen Querschnitt durch alle drei Komponenten des Sets. Das plattenförmige Grundelement 1 verfügt über wenigstens zwei, vorliegend vier Durchbohrungen 2. In jede der Durchbohrungen 2 ist ein Zuganker 3 mit seinem ersten Ende 4 setzbar. Das zweite Ende 7 des Zugankers 3 ist vorliegend mit einem Gewinde 6 versehen. Mit dem Gewinde 6 verschraubbar ist ein Gewindeelement 5 in Form einer Gewindehülse mit einem im Kopf vorgesehenen Werkzeugansatz 10, beispielsweise ein Innensechskant.

Die Besonderheit der Verbindung zwischen Zuganker 3 und Grundelement 1 ist in Figur 2 veranschaulicht. In eine Durchbohrung 2 in dem Grundelement 1 ist ein erstes Ende 4 eines Zugankers 3 gesetzt. Die Durchbohrung 2 und das erste Ende 4 des Zugankers 3 sind nun so ausgebildet, dass das Ende 4 in der Durchbohrung 2 verriegelbar ist, wobei der Zuganker 3 im verriegeltem Zustand eine Schwenkbewegung ausführen kann, wie dies in Figur 2 durch die gestrichelten Lagen des Zugankers 3 veranschaulicht ist, wobei vorliegend ein Verschwenken um ± 10° um die Nulllage gezeigt ist, d. h. der Lage, in welcher der Zuganker 3 senkrecht auf dem plattenförmigen Grundelement 1 steht. Diese Verschwenkbarkeit kommt bei der Implantation nur indirekt zum Tragen, wie anhand der Figur 5 weiter unten näher erläutert wird.

Die dem Knochen zuzuwendende Fläche des Grundelementes 1 ist vorliegend abschnittsweise mit einer offenmaschigen, dreidimensionalen Raumnetzstruktur 8 versehen, in welche und durch welche hindurch sich dann Knochentrabekel organisieren und so für eine dauerhafte Sekundärfixation sorgen. Eine vergleichbare Struktur ist vorliegend an der dem Knochen zuzuwendenden Fläche des Gewindeelementes 5 vorgesehen (Figur 1).

Figur 3 zeigt eine Aufsicht auf das plattenförmige Grundelement 1 mit den vier Durchbohrungen 2. Ferner ist die vergrößerte Einzelheit Z zu erkennen, aus der heraus eine spezielle Kontur der Durchbohrungen 2 ersichtlich wird. Diese ist notwendig, um einerseits eine Verriegelung mit dem Zuganker 3 zu gestatten, andererseits aber auch, um trotz Verriegelung eine gewisse Verschwenkbarkeit zu erhalten. Der Pfeil A deutet die Einsatzrichtung des ersten Endes 4 eines Zugankers 3 in die Durchbohrung 2 an. Der Pfeil B deutet dann die Bewegung des Zugankers 3 in der Durchbohrung 2 bei der Verriegelung an. Vorliegend bilden die ersten Enden 4 des Zugankers 3 mit den Durchbohrungen 2 eine Art Bajonettverschluss aus.

Die Verschwenkbarkeit des ersten Endes 4 des Zugankers 3 in der Durchbohrung 2 in dem Grundelement 1 soll in Figur 4 angedeutet sein. Trotz Formenschlusses gegen eine weitere Rotation in der verriegelten Lage ist es möglich, den Zuganker 3 in der Durchbohrung 2 zu verschwenken, wie dies in Figur 2 angedeutet ist.

Die Wirkungsweise des Sets bzw. des daraus erstellten Osteosynthesimplantates ist in Figur 5 angedeutet. Diese zeigt im Querschnitt das Osteosyntheseimplantat in situ am Knochen 11 zur Stabilisierung der Fraktur 12.

Das Grundelement 1 liegt an einer Seite des zu stabilisierenden Knochens 11 an. Es wird dann mit einer Spannzange (nicht dargestellt) dort unterklemmt. Sie dient in dieser Lage als Bohrlehre, um vorliegend vier Bohrungen in den Knochen einzubringen, und zwar in unterschiedlicher Winkellage, in Abhängigkeit von den Formen und Gegebenheiten des Knochens. Nach Einbringung der Durchbohrungen im Knochen 11 wird die Einheit aus Zuganker 3 mit darauf aufgeschraubtem Gewindeelement 5 in die jeweilige Durchbohrung in den Knochen gesetzt, bis dessen erstes Ende in den Bereich der Durchbohrung 2 im Grundelement 1 kommt. Eine weitere Drehung des Gewindeelementes 5 führt zu der Verriegelung des Zugankers 3 in der Durchbohrung 2. Es ist einsichtig, dass Voraussetzung für das Einsetzen der Zuganker 3 in die Bohrungen unterschiedlicher Orientierung bei der Implantation die weiter oben näher erläuterte Verschwenkbarkeit des Zugankers 3 in der Durchbohrung 2 ist.

Das Ergebnis ist ein spannungsfreier Sitz des Implantates im und an dem Knochen 11, so dass insofern keine weiteren Belastungen auf den Knochen 11 und auch nicht auf das Implantat einwirken, die nicht schon vorher erkannt werden können. Ein weiteres Anziehen der Gewindeelemente 5 führt zur Erhöhung der Zugkräfte in den Zugankern 3, was zu einer höheren Stabilisierung der Fraktur 12 führen kann.

Das Grundelement 1, die Zuganker 3 sowie die Gewindeelemente 5 bestehen bevorzugt aus einem körperverträglichen Metall, um insofern den auftretenden Belastungen Stand halten zu können.

## Patentansprüche

1. Set zur Erstellung eines Osteosyntheseimplantates, aufweisend
- ein plattenförmiges Grundelement (1) mit wenigstens zwei Durchbohrungen (2),
- wenigstens zwei stabförmige Zuganker (3), die mit ihrem einen ersten Ende (4) in die Durchbohrungen (2) setzbar sind, sowie
- wenigstens zwei Gewindeelemente (5), die mit den mit jeweils einem Gewinde (6) versehenen zweiten Enden (7) der wenigstens zwei Zuganker (3) verschraubbar sind,
**dadurch gekennzeichnet, dass** die Durchbohrungen (2) und die ersten Enden (4) der Zuganker (3) derart ausgebildet sind, dass die Enden (4) in den Durchbohrungen (2) verriegelbar sind, wobei die Zuganker (3) im verriegeltem Zustand eine Schwenkbewegung ausführen können und dass die jeweils mit ihren zweiten Enden mit den wenigstens zwei Fewindeelementen verschraubten Zuganker in die Durchbohrungen setzbar und verriegelbar sind.

2. Set nach Anspruch 1, bei dem die Zuganker (3) im in den Durchbohrungen (2) verriegelten Zustand eine Schwenkbewegung um die Nulllage, in der die Zuganker (3) senkrecht auf dem plattenförmigen Grundelement (1) stehen, um ± 20° ausführen können.

3. Set nach Anspruch 1 oder 2, bei dem die Zuganker (3) Gewindehülsen und die Gewindeelemente (5) Schrauben sind.

4. Set nach Anspruch 1 oder 2, bei dem die Zuganker (3) an ihren zweiten Enden (7) als Gewindespindeln und die Gewindeelemente (5) als Gewindehülsen ausgebildet sind.

5. Set nach einem der Ansprüche 1 bis 4, bei dem die ersten Enden (4) der Zuganker (3) mit den Durchbohrungen (2) im plattenförmigen Grundelement (1) eine Art Bajonettverschluss ausbilden.

6. Set nach einem der Ansprüche 1 bis 5, bei dem die dem Knochen zuzuwendende Fläche des Grundelements (1) zumindest abschnittsweise mit einer offenmaschigen, dreidimensionalen Raumnetzstruktur (8) versehen ist.

7. Set nach einem der Ansprüche 1 bis 6, bei dem die dem Knochen zuzuwendenden Flächen der Gewindeelemente (5) zumindest teilweise mit einer offenmaschigen, dreidimensionalen Raumnetzstruktur (9) versehen ist.

8. Set nach einem der Ansprüche 1 bis 7, bei dem das Grundelement (1), die Zuganker (3) und die Gewindeelemente (5) aus körperverträglichem Metall bestehen.

## Claims

1. A set for creating an osteosynthesis implant, comprising
- a plate-like base element (1) with at least two through-bores (2),
- at least two rod-like tension rods (3) which with their first end may be applied into the through-bores (2), as well as
- at least two threaded elements (5), which may be screwed to the two ends (7) of the at least two tension members (3), said ends being provided in each case with a thread (6),
**characterised in that** the through-bores (2) and the first ends (4) of the tension members (3) are designed in a manner such that the ends (4) may be locked in the through-bores (2), wherein the tension members (3) may execute a pivoting movement in the locked condition, and that the tension members screwed in each case with their second ends to the at least two threaded elements, may be applied into the through-bores and locked.

2. A set according to claim 1, with which the tension members (3) in the condition locked in the through-bores (2), may execute a pivoting movement by ± 20° about the zero position, in which the tension members (3) stand perpendicularly on the plate-like base element (1).

3. A set according to claim 1 or 2, with which the tension members (3) are threaded sleeves and the threaded elements (5) are screws.

4. A set according to claim 1 or 2, with which the tension members (3) at their second ends (7) are designed as threaded spindles and the threaded elements (5) as threaded sleeves.

5. A set according to one of the claims 1 to 4, with which the first ends (4) of the tension members (3), form a type of bayonet closure with the through-bores (2) in the plate-like base element (1).

6. A set according to one of the claims 1 to 5, with which the surface of the base element (1) which are to face the bone, is provided at least in sections with an open-meshed, three-dimensional spatial net structure (8).

7. A set according to one of the claims 1 to 6, with which the surfaces of the threaded elements (5) which are to face the bone, are a least partly provided with an open-meshed, three-dimensional spatial net structure (9).

8. A set according to one of the claims 1 to 7, with which the base element (1), the tension member (3) and the threaded elements (5) consist of body-compatible metal.

## Revendications

1. Set de fabrication d'un implant d'ostéosynthèse, comportant
- un élément de base en forme de plaque (1) qui comprend au moins deux trous traversants (2),
- au moins deux tirants en forme de tige (3) qui peuvent être placés dans les trous traversants (2) par leur première extrémité (4), ainsi que
- au moins deux éléments filetés (5) qui peuvent être vissés avec les secondes extrémités (7) des tirants (3), au moins au nombre de deux, munies respectivement d'un filetage (6),
**caractérisé en ce que** les trous traversants (2) et les premières extrémités (4) des tirants (3) sont conçus de façon telle que les extrémités (4) peuvent être verrouillées dans les trous traversants (2), les tirants (3) à l'état verrouillé pouvant exécuter un mouvement pivotant, et **en ce que** les tirants vissés respectivement par leurs secondes extrémités avec les éléments filetés, au moins au nombre de deux, peuvent être placés et verrouillés dans les trous traversants.

2. Set selon la revendication 1, dans lequel les tirants (3), à l'état verrouillé dans les trous traversants (2), peuvent exécuter un mouvement pivotant de ± 20° autour de la position zéro, dans laquelle les tirants (3) sont placés verticalement sur l'élément de base en forme de plaque (1).

3. Set selon la revendication 1 ou 2, dans lequel les tirants (3) sont des douilles filetées et les éléments filetés (5) sont des vis.

4. Set selon la revendication 1 ou 2, dans lequel les tirants (3) sont conçus au niveau de leurs secondes extrémités (7) sous forme de broches filetées et les éléments filetés (5) sous forme de douilles filetées.

5. Set selon l'une des revendications 1 à 4, dans lequel les premières extrémités (4) des tirants (3) forment avec les trous traversants (2) dans l'élément de base en forme de plaque (1) un genre de raccord à baïonnette.

6. Set selon l'une des revendications 1 à 5, dans lequel la surface de l'élément de base (1) orientée vers l'os est munie, au moins sur des segments, d'une structure spatiale maillée (8) en trois dimensions et à mailles ouvertes.

7. Set selon l'une des revendications 1 à 6, dans lequel les surfaces des éléments filetés (5) orientées vers l'os sont munies au moins partiellement d'une structure spatiale maillée (9) en trois dimensions et à mailles ouvertes.

8. Set selon l'une des revendications 1 à 7, dans lequel l'élément de base (1), les tirants (3) et les éléments filetés (5) se composent d'un métal compatible avec le corps.
